# EUROPEAN PATENT APPLICATION

(11) **EP 2 548 539 A1**
(43) Date of publication of application: **23.01.2013**
(21) Application number: 11756010.2
(22) Date of filing: 07.02.2011
(51) Int. Cl.: A61F 13/15, A61F 13/49

(54) **ABSORPTIVE ARTICLE MANUFACTURING DEVICE**

(30) Priority: 19.03.2010 JP 2010064065
(71) Applicant: Unicharm Corporation, Ehime 799-0111 (JP)
(72) Inventor: OBA, Kenji, Kanonji-shi Kagawa 769-1602 (JP)
(74) Representative: Peter, Julian
(86) International application number: PCT/JP2011/052971
(87) International publication number: WO 2011/114820

(57) **Abstract**

An absorbent article manufacturing device is formed by mutually combining a plurality of processing units (2). Spacers (6) are provided on the bottom of the processing units (2) to form a lower space (SL) between an installation plane (PI) where the processing units (2) are installed and the processing units (2). Product-related articles such as materials are moved within the lower space (SL). Alternatively, manufacturing-related articles such as electrical wiring are arranged within the lower space (SL).

## Description

### Technical Field

The present invention relates to an absorbent article manufacturing device.

### Background Art

An absorbent article manufacturing device is known that is formed by mutually combining a plurality of processing units (see PLT 1). In this manufacturing device, the processing units are dimensioned that allow them to be housed in a shipping container, thereby enabling the manufacturing device to be easily relocated.

### Citation List

### Patent Literature

PLT 1: Japanese Unexamined Patent Publication No. 2009-39441

### Summary of Invention

### Technical Problem

However, in the case processing units are dimensioned as described for the aforementioned manufacturing device, the processing space of the processing units is not necessarily large. As a result, it is difficult to secure space for product-related articles such as materials, intermediate products and finished products, and manufacturing-related articles such as adhesive lines or electrical wiring.

In order to solve this problem, it may be considered to utilize space above and below the processing units. However, if a non-woven fabric web is transported in the upper space, for example, the transport distance of the non-woven fabric web increases resulting in the risk of snaking of the non-woven fabric web. In addition, if an adhesive line, for example, is arranged in the upper space, it is necessary to increase the feed pressure of the adhesive in order to feed the adhesive to the upper space, thereby resulting in the risk of the appropriate adhesive pressure deviating from the proper value. Alternatively, in the case a problem occurs in materials and so forth, a worker is required to acquire a ladder and the like and climb up the ladder to perform work, thereby resulting in the risk of preventing maintenance from being performed easily and rapidly.

### Solution to Problem

According to the present invention, an absorbent article manufacturing device is provided that is formed by mutually combining a plurality of processing units, wherein spacers are provided on the bottom of at least one processing unit to form a lower space between the installation plane on which the processing unit is installed and the processing unit, and product-related articles are moved or manufacturing-related articles are arranged within the lower space.

### Advantageous Effects of Invention

Space can be secured for product-related articles or manufacturing-related articles while ensuring favorable manufacturing of absorbent articles.

### Brief Description of the Drawings

FIG. 1 is a schematic front view of an absorbent article manufacturing device.
FIG. 2 is a schematic plan view of an absorbent article manufacturing device.
FIG. 3 is a front view of a processing unit.
FIG. 4 is a side view of a processing unit.
FIG. 5 is a plan view of a processing unit.
FIG. 6 is a bottom view of a processing unit.
FIG. 7 is a partially enlarged view of a processing unit.
FIG. 8 is a schematic diagram showing the flow of product-related articles.

### Description of Embodiments

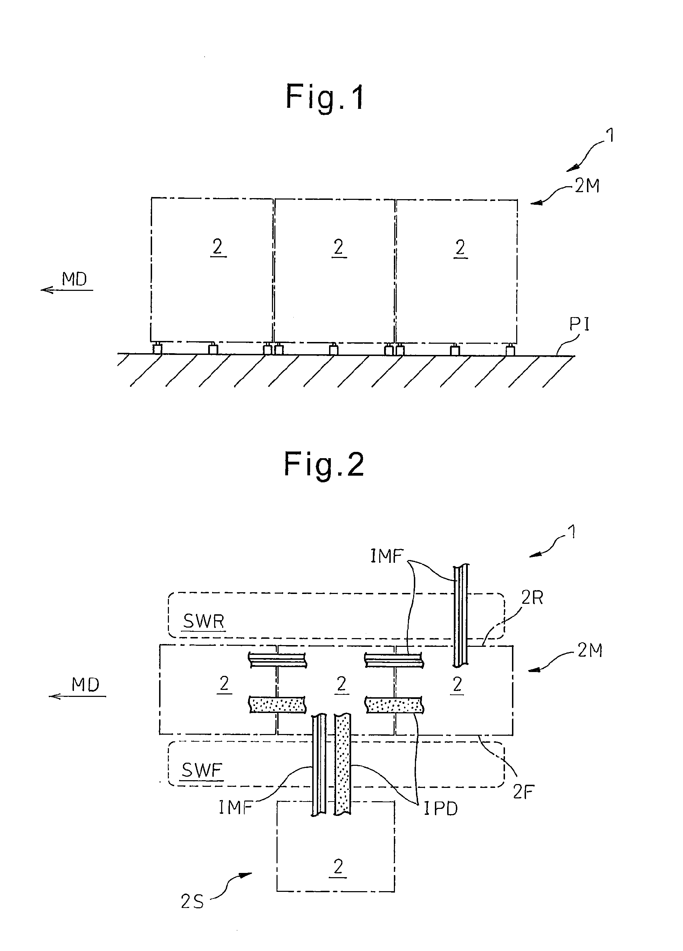

With reference to FIGS. 1 and 2, an absorbent article manufacturing device 1 is provided with a plurality of processing units 2, and the manufacturing device 1 is formed by mutually combining these processing units. In this connection, examples of the absorbent article include sanitary napkins, panty liners, urinary incontinence pads, diapers or the like.

In the embodiment shown in FIGS. 1 and 2, the manufacturing device 1 is provided with a main unit assembly 2M comprised of at least one processing unit 2, and this main unit assembly 2M is comprised of, for example, three processing units 2 arranged in a row in a machine direction MD and installed on an installation plane PI.

Worker movement spaces SWF and SWR are provided respectively adjacent to a front surface 2F and back surface 2B of the main unit assembly 2M and/or the processing units 2. A worker accesses the processing units 2 from the front surface 2F or back surface 2R by moving through the worker movement spaces SWF and SWR.

The manufacturing device 1 is further provided with a subunit assembly 2S comprised of at least one processing unit 2, and the subunit assembly 2S is arranged at a distance from the main unit assembly 2M by a distance equal to the worker movement spaces SWF and SWR. In the embodiment shown in FIG. 2, the subunit assembly 2S is comprised of a single processing unit 2, and is arranged in front of the main unit assembly 2M at a distance from the main unit 2M equal to the worker movement space SWF.

In the processing units 2, processing such as transporting, unwinding or winding, joining, sealing, temporarily holding, cutting, overlapping, compressing or stretching, folding, heating or cooling, or applying an adhesive or pressure-sensitive adhesive, is carried out on product-related articles such as materials, intermediate products or finished products, to thereby produce an absorbent article. In this case, product-related articles IPR are respectively moved between the processing units 2 and between the main unit assembly 2M and the subunit assembly 2S. Note that materials include non-woven fabric, plastic film, pulp and fibrous or sheet-like elastic bodies, and the like.

In addition, manufacturing-related articles such as lines for fluids such as adhesive, hydraulic fluid or air and electrical wiring for power lines or signal lines, are arranged so as to extend between the processing units, between the main unit assembly 2M and the subunit assembly 2S, or between the processing units 2 and the outside.

Each processing unit 2 is dimensioned enabling it to be housed in a shipping container that complies with Japanese Industrial Standards (JIS) or the International Organization for Standardization (ISO). As a result, the processing units 2 can be moved while housed in a shipping container without having to disassemble the processing units 2. Thus, the manufacturing device 1 can be easily relocated.

Examples of shipping containers that can be used for housing the processing units 2 include 20 ft dry containers or 40 ft high cube containers. The outer dimensions of a 20 ft dry container are 20' (6096 mm) long × 8' (2438 mm) wide × 8'6" (2591 mm) high, while the outer dimensions of a 40 ft high cube container are 40' (12192 mm) long × 8' (2438 mm) wide × 9'6" (2895 mm) high.

When considering that the processing units 2 are packaged before housing in a container, the maximum possible dimensions of the processing units 2 in the case of using a 20 ft container are as follows:

| | L (mm) × W (mm) × H (mm) |
|---|---|
| Total weight of 3 tons or less | 5680 × 2070 × 1850 |
| Total weight of 3 to 6 tons | 5600 × 2030 × 1750 |
| Total weight of 6 tons or more | 5540 × 2010 × 1600 |

Similarly, the maximum possible dimensions of the processing units 2 in the case of using a 40 ft high cube container are as follows:

| | L (mm) × W (mm) × H (mm) |
|---|---|
| Total weight of 3 tons or less | 11810 × 2070 × 2190 |
| Total weight of 3 to 6 tons | 11730 × 2030 × 2120 |
| Total weight of 6 tons or more | 11670 × 2010 × 1970 |

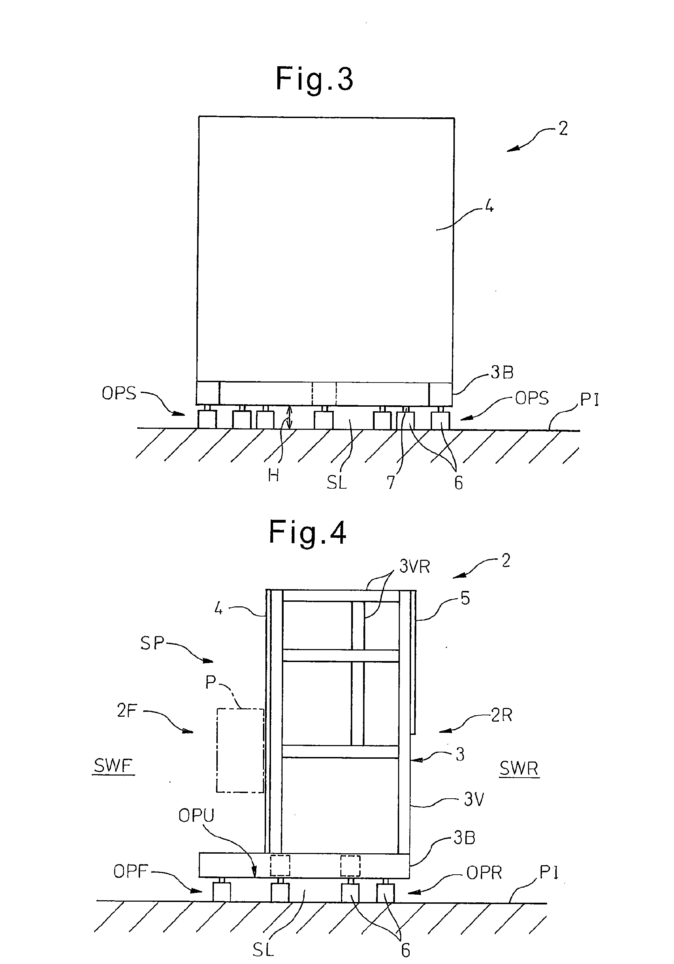

With reference to FIGS. 3 to 6, each of the processing units 2 is provided with a frame 3. The frame 3 includes a base frame 3B located in the horizontal plane at the bottom of the processing unit 2, and a vertical frame 3V extending vertically from the base frame 3B. Note that, in the drawings, 3VR indicates reinforcing members that reinforce the vertical frame 3V.

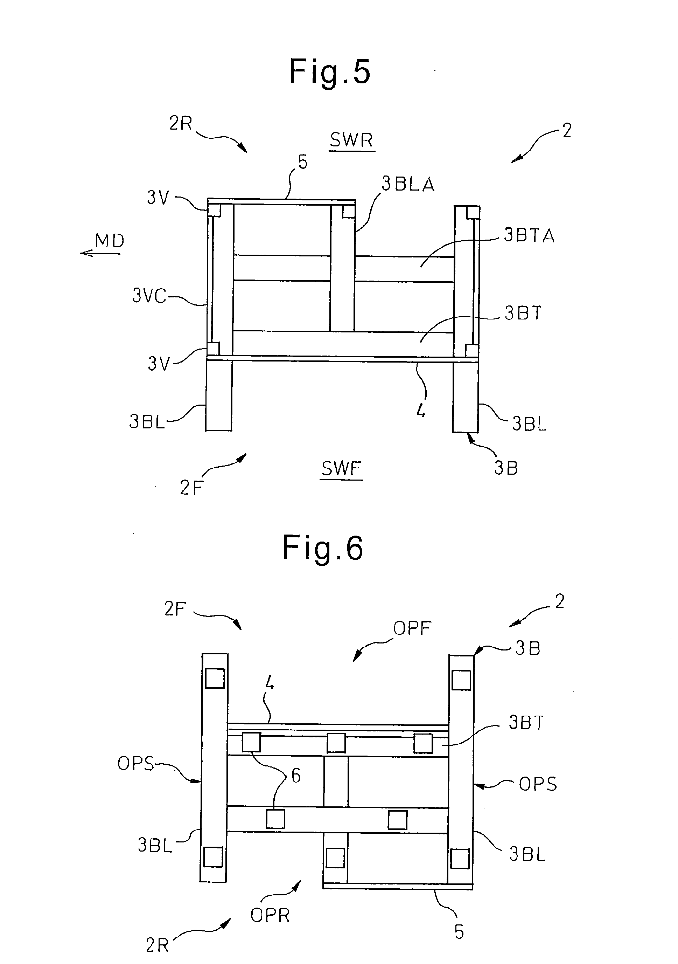

As shown in FIG. 5 in particular, the base frame 3B includes an H-shaped portion comprising a pair of longitudinal portions 3BL extending forward and backward perpendicular to the machine direction MD, and a transverse portion 3BT extending to the left and right parallel to the machine direction MD, and further includes an additional longitudinal portion 3BLA facing towards the back from the transverse portion 3BT and an additional transverse portion 3BTA mutually connecting the pair of longitudinal portions 3BL farther towards the back than the transverse portion 3BT.

A panel 4 extending in the vertical direction is provided within a processing space SP defined above the base frame 3, and the panel 4 is supported by the vertical frame 3V and the transverse portion 3BT of the base frame 3 of the processing unit 2. As shown in FIG. 4, a processor P is cantilever supported by the front of the panel 4. In this case, a worker can perform maintenance, for example, by accessing the processor P from the worker movement space SWF. Note that the processor P is omitted from FIGS. 3, 5 and 6.

On the other hand, behind the processing unit 2, an electrical control panel 5 for electrically controlling the manufacturing device 1 is supported by the vertical frame 3V. A worker can operate the electrical control panel 5 by accessing the electrical control panel 5 from the worker movement space SWR. In this case, as can be understood from FIGS. 4 and 5, the electrical control panel 5 is provided at a distance away from the panel 4. In addition, the electrical control panel 5 does not extend over the entire width of the processing unit 2, but only over a portion thereof. As a result, a worker is able to easily access the back of the panel 4. A pulley for driving a roller or a motor for rotating and driving the pulley, for example, are provided on the back of the panel 4. Thus, maintenance can be performed easily on the pulley, motor and the like.

As is shown in FIGS. 3, 4 and 6, block-shaped, for example, spacers 6 are provided on the bottom of the base frame 3B, namely the processing unit 2. As a result, a lower space SL is formed between the processing unit 2 and the installation plane PI.

This allows that product-related articles are moved within the lower space SL. In addition, manufacturing-related articles can be arranged within the lower space SL. In this case, since the heights at which the product-related articles and manufacturing-related articles are positioned are low, a worker can easily access the product-related articles and manufacturing-related articles, and maintenance can be performed easily. There is also no need to increase the feed pressure of an adhesive and the like. Note that the spacers 6 need not be provided for all of the processing units 2. Namely, the spacers 6 are provided for at least one of the processing units 2.

The height H of the lower space SL is set to, for example, 185 mm. Note that, when considering the dimensions of product-related articles to be moved within the lower space SL or the dimensions of manufacturing-related articles to be arranged within the lower space SL, the height H of the lower space SL is preferably 50 mm or more. In this manner, the lower space SL differs from a simple gap formed below the processing unit 2.

Bolts 7 are fixed to the bottom of the base frame 3B, and the spacers 6 are attached to the processing unit 2 by engaging with these bolts 7. As a result, the locations of the spacers 6 relative to the processing unit 2 can be adjusted, thereby enabling the size of the lower space SL to be adjusted. In this case, the adjusted amount of the spacers 6 can be made to be ±25 mm.

The peripheral surface of the lower space SL respectively includes an open front portion OPF, an open rear portion OPR and open side portions OPS, and the lower space SL and the outside space are mutually communicated through the open front portion OPF, open rear portion OPR and open side portions OPS. Thus, product-related articles and manufacturing-related articles are able to reach the lower space SL from the outside space or are able to reach the outside space from the lower space SL by passing through the open front portion OPF, the open rear portion OPR and the open side portions OPS.

In addition, the upper surface of the lower space SL also includes an open upper portion OPU that is open, and the lower space SL and the processing space SP are mutually communicated through this open upper portion OPU. Thus, product-related articles and manufacturing-related articles are able to reach the lower space SL from the processing space SP or are able to reach the processing space SP from the lower space SL by passing through the open upper space OPU. With respect to this point, the base frame 3 can also be interpreted as being constituted so as to form the open upper portion OPU.

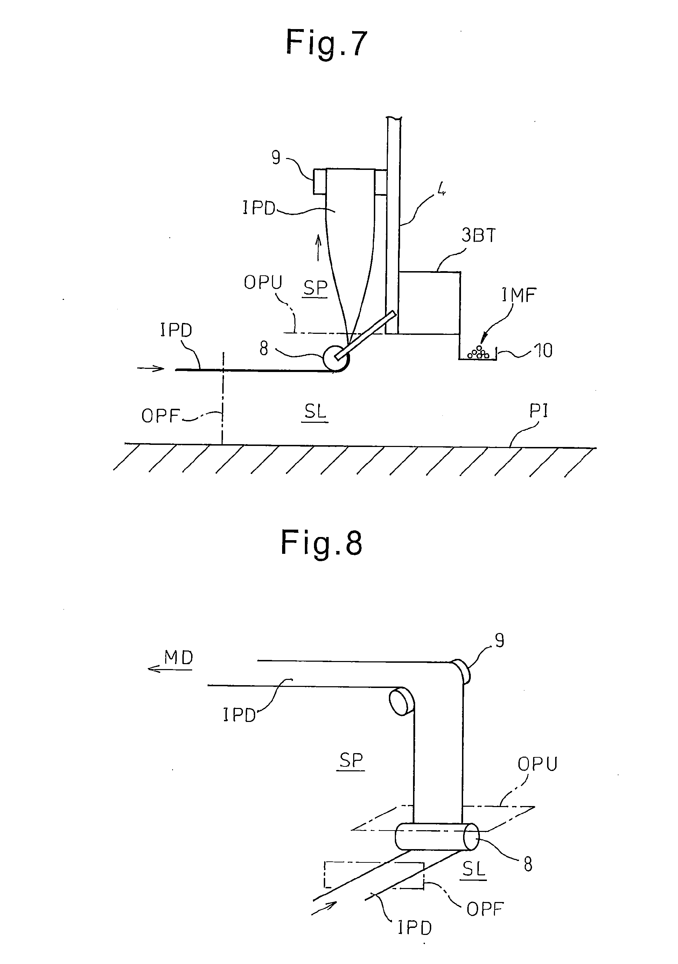

In the embodiment shown in FIGS. 7 and 8, guide rollers 8 and 9 are removably provided on the panel 4. In this case, a product-related article IPD, for example, in the form of a web, reaches the lower space SL through the open front portion OPF and is transported to the lower space SL. The product-related article IPD is then transported upward guided by the guide roller 8, and reaches the processing space SP through the open upper portion OPU. The product-related article IPD is then simultaneously twisted 90 degrees and is transported in the horizontal direction and/or machine direction MD guided by the guiding roller 9. As a result, since the product-related article IPD can be moved to substantially directly beneath a processor within the processing space SP, the transport pathway of the product-related article IPD can be shortened and snaking of the product-related article IPD can be suppressed.

Note that, in the embodiment shown in FIGS. 7 and 8, the product-related article IPD traverses the worker movement space SWF (see FIG. 2). However, since the height at which the product-related article IPD is positioned is low, a worker can easily straddle the product-related article IPD. Alternatively, providing a stepladder that straddles the product-related article IPD prevents the product-related article IPD from impairing work performed by a worker.

In addition, in the embodiment shown in FIG. 7, a holder 10 is attached to the transverse portion 3BT, and the product-related article IMF is housed within this holder 10. As a result, the product-related article IMF can be held within the lower space SL at a distance away from the installation plane PI. As a result, in the case dust is present or a liquid has spilled on the installation plane PI, the product-related article IMF can be protected from this dust or liquid, and the lower space SL can be cleaned easily. In this case, electrical wiring of the product-related article IMF may extend upward through the open upper portion OPU to reach the electrical control panel 5.

The guide rollers 8 and 9 may also be attached to the base frame 3 or the spacers 6. In addition, the holder 10 may also be attached to the panel 4 or the spacers 6. Note that the spacers 6 are not shown in FIGS. 7 and 8 to facilitate legibility.

### Reference Signs List

- 1: Manufacturing device
- 2: Processing unit
- 6: Spacers
- SL: Lower space

## Claims

1. An absorbent article manufacturing device formed by mutually combining a plurality of processing units; wherein,
spacers are provided on the bottom of at least one processing unit to form a lower space between the installation plane on which the processing unit is installed and the processing unit, and product-related articles are moved or manufacturing-related articles are arranged within the lower space.

2. The manufacturing device according to claim 1, wherein the processing unit is dimensioned enabling it to be housed in a shipping container that complies with Japanese Industrial Standards or the International Organization for Standardization together with the spacers.

3. The manufacturing device according to claim 1 or 2, wherein the peripheral surface of the lower space includes open peripheral portions, and the lower space and outside space are mutually communicated through the open peripheral portions.

4. The manufacturing device according to any of claims 1 to 3, wherein the upper surface of the lower space includes an open upper portion, and the lower space and a processing space of the processing unit are mutually communicated through the open upper portion.

5. The manufacturing device according to claim 4, wherein a guide roller is further provided for guiding a product-related article that moves between the lower space and the processing space of the processing unit.

6. The manufacturing device according to any of claims 1 to 5, wherein a holder is further provided that holds a product-related article within the lower space at a distance away from the installation plane.

7. The manufacturing device according to any of claims 1 to 6, wherein a base frame of the processing unit includes an H-shaped portion comprising a pair of longitudinal portions and a transverse portion that mutually connects these longitudinal portions, and a panel extending in the vertical direction is supported by the transverse portion.

8. The manufacturing device according to claim 7, wherein an electrical control panel is provided behind the panel at a distance away from the panel.
